# EUROPEAN PATENT APPLICATION

(11) **EP 4 650 378 A1**
(43) Date of publication of application: **19.11.2025**
(21) Application number: 25176486.6
(22) Date of filing: 14.05.2025
(51) Int. Cl.: C08G 18/16, C08G 18/18, C08G 18/20, C08G 18/24, C08G 18/36, C08G 18/48, C08G 18/66, C08G 18/76

(54) **METHOD OF OBTAINING POLYOL FROM VEGETABLE OIL, POLYOL OBTAINED BY THIS METHOD AND ITS USE FOR POLYURETHANE FOAM**

(30) Priority: 16.05.2024 PL 44858324
(71) Applicant: PCC ROKITA Spolka Akcyjna, 56-120 Brzeg Dolny (PL)
(72) Inventor: Smagowicz, Anna, 56-120 Brzeg Dolny (PL); Plocieniak, Miroslaw, 51-531 Wroc aw (PL); Pawlik, Henryk, 31-875 Krakow (PL); Wojdyla, Henryk, 56-100 Wolow (PL); Kacperski, Michal, 54-613 Wroclaw (PL); Makula, Lukasz, 51-114 Wroclaw (PL); Salasa, Michal, 56-120 Brzeg Dolny (PL)

(57) **Abstract**

The invention relates to a method for obtaining a natural polyol (NOP) from a vegetable oil with a reduced content of unsaturated bonds, using a reduced amount of nucleophile and double purification. The invention also relates to a polyol obtained by such a method and the use of such a natural polyol for the production of polyurethane foam with favorable smell and mechanical properties.

## Description

### Field of invention

The invention relates to a method for obtaining a natural polyol (NOP) from a vegetable oil with a reduced content of unsaturated bonds, using a reduced amount of nucleophile and double purification. The invention also relates to a polyol obtained by such a method and the use of such a natural polyol for the production of polyurethane foam with favorable smell and mechanical properties.

### State of the art

Polyols are materials for the production of polyurethane foams. Such foams are characterized by a wide range of applications in many industries such as the furniture, automotive and construction industries. These materials can be divided, due to their properties, into two main groups: flexible foams and rigid foams. Until recently, mainly petroleum-derived raw materials were used for the production of polyols, and the products obtained from them are unfortunately characterized by a very long decomposition time and an adverse effect on the environment. The growing need to take care of environmental protection aspects increases the tendency to replace petrochemical raw materials with renewable raw materials, also in the polyurethane industry. Polyols from vegetable oils have been defined as one of the forms of products based on renewable sources.

Work on the use of vegetable oils for the synthesis of polyols has been successfully conducted since the 1970s. It concerns primarily the following oils: soybean (SO), rapeseed (RO), sunflower, palm and castor. These oils differ in the composition of fatty acids, which consequently leads to differences in the applications of the oils themselves and the products obtained from them.

Polyols are synthesized from vegetable oils mainly by:
- hydroformylation and reduction of aldehyde groups,
- epoxidation and subsequent opening of oxirane rings,
- ozonolysis and hydrogenation,
- transesterification,
- microbiological conversion.

EP4053111A1 presents a method for producing biopolyol from vegetable oil using the epoxidation process of double bonds in the presence of a catalyst. Rapeseed oil with an iodine value of 105 - 120 g/100g is subjected to the process of epoxidation of double bonds using an oxidation system consisting of 35% dihydrogen peroxide and 99% glacial acetic acid in the presence of an acid catalyst, e.g. sulfuric acid (VI) (see claim 2), wherein the content of 35% dihydrogen peroxide per mole of double bonds of rapeseed oil is from 1.0 to 5.0 moles, the content of 99% glacial acetic acid per mole of double bonds of rapeseed oil is from 0.1 to 1.0 moles, and the content of the acid catalyst is from 1.0 to 6.0 gm/m3 of concentrated acid catalyst per 100 gm/m³ of the reaction mixture, and the epoxidation process is carried out at a temperature of 35 to 75°C with the selectivity of the epoxidation reaction above 70% and the conversion of the reaction epoxidation above 90%; purification of the residue of unreacted dihydrogen peroxide 35% and acetic acid 99% by known methods; drying by vacuum distillation at a temperature of 80 - 120°C using a vacuum of 0.6 - 0.8 mbar until the water content is below 0.1% m/m, preferably 0.06% m/m. Epoxidized rapeseed oil is subjected to the epoxy ring opening process with a nucleophile in the presence of an acid catalyst, in which the purified epoxy oil with an epoxide value of 0.1-0.2 mol /100 g of epoxy oil is subjected to a single-stage epoxy ring opening reaction with a nucleophile in a ratio of 1-2 moles of nucleophile to moles of epoxy groups of the purified epoxy oil, in the presence of an acid catalyst in the amount of 2-5 g /1 mole of epoxy groups. The crude biopolyol obtained in this way is subjected to the naturalization process, water washing and distillation process. The obtained polyol was used to produce polyurethane foam with diphenylmethane diisocyanate (MDI) in the presence of a silicone surfactant, a mixture of an amine catalyst in the form of PMDTA and N,N-dimethylcyclohexylamine, and water was used as the foaming agent.

Also from document PL206376B1 there is known a method of obtaining a polyol component intended for the synthesis of flexible, polyurethane porous materials in a two-stage process consisting of epoxidation of vegetable oil by means of an oxidizing system consisting of acetic acid and a hydrogen peroxide solution in the first stage and reacting the oxidized oil with ethylene glycol in the second stage. Rapeseed oil is subjected to an epoxidation reaction in an oxidizing system containing a 30% hydrogen peroxide solution, at a molar ratio of acetic acid to the content of unsaturated bonds in the oil of 0.3 to 1.0 and a molar ratio of hydrogen peroxide to the content of unsaturated bonds in the oil of 1.0 to 1.4, and the reaction is carried out in a solvent, preferably toluene, in the presence of a catalyst in the form of sulfuric acid (VI), introduced in an amount of 3 to 12 parts by weight per 100 parts by weight of the total mass of acetic acid and hydrogen peroxide used in the reaction, at an epoxidation reaction temperature of 50 to 70°C for a period of 4 to 12 hours. In the second stage, the oxidized oil is reacted with ethylene glycol used in a stoichiometric amount relative to the content of epoxy groups in the oil, in the presence of an acid catalyst, in an amount of 0.8 to 2 g, preferably 1 g of acid per 1 mole of epoxy groups in the oil, the reaction being carried out at a temperature of 100 to 120°C for a period of 3 to 24 hours.

Document PL233221B1 discloses a method for obtaining a biopolyol intended for the synthesis of polyurethane-polyisocyanurate foams, proceeding in a two-stage process consisting in the epoxidation of vegetable oil using a hydrogen peroxide solution in the presence of acetic acid and a catalyst in the first stage and the opening reaction of oxirane rings using glycol in the presence of a catalyst in the second stage. The epoxidation reaction is carried out on raw white mustard oil, evening primrose or milk thistle oil in a system containing: 30-35% hydrogen peroxide solution in a molar ratio to the content of unsaturated bonds in the oil of 0.5 to 2.0; 99.5 - 100% acetic acid in a molar ratio to the content of unsaturated bonds in the oil of 0.8 to 1.2 and 96% sulfuric acid (VI) as a catalyst in a molar ratio to the content of unsaturated bonds in the oil of 0 to 0.08. The reaction is carried out for 3 to 6 hours at a temperature of 40°C during hydrogen peroxide dosing and 60°C after its complete introduction. In the second stage, the obtained epoxidized oil is reacted with thiodiethylene glycol in a stoichiometric ratio to the content of epoxy groups in the presence of 96% sulfuric acid (VI) in a molar ratio to the content of epoxy groups of 0.01 to 0.07, the reaction is carried out at a temperature of 120°C until complete substitution of epoxy groups for 4 to 8 hours.

The document H. Pawlik, A. Prociak, J. Pielichowski, "Synthesis of polyols from palm oil intended for obtaining flexible polyurethane foams", Technical Journal. Chemistry, 2009, no. 1-Ch, issue 4, pp. 111 - 117, presents a method for producing polyols for obtaining flexible polyurethane foams from palm oil. The synthesis was carried out using a two-stage method. In the first stage, double bonds in unsaturated fatty acids were oxidized. The oxidizing agent was acetic peracid formed in situ as a result of the action of hydrogen peroxide on acetic acid in the presence of sulfuric acid. As a result of the oxidation reaction, epoxidized oil was obtained, from which acids were then washed out with water. The second stage of the synthesis consisted in opening oxirane rings in epoxidized palm oils. As a result of the action of the ring-opening agent in the form of diethylene glycol (DEG) in the presence of sulfuric acid, polyols with different values of hydroxyl values were obtained. The content of hydroxyl groups in the polyol can be planned already at the stage of oil oxidation by ensuring appropriate conditions for the reaction. Using the presented two-stage synthesis, polyols with the desired values of the hydroxyl value can be obtained in various ways. Namely, by opening a specific number of epoxy groups, by acting on the fully oxidized oil with a calculated amount of the opening agent or by oxidizing an appropriate number of double bonds and then completely opening the oxirane rings.

There are known from Polish and English patent specifications (PL198322, GB739609, GB769127 and GB790063) methods of obtaining epoxidized vegetable oils which are most often carried out in the system: hydrogen peroxide and aliphatic carboxylic acid, in the presence of an acid catalyst. The oxidizing agent in such a case is an organic peracid formed in situ in the reaction of acid with hydrogen peroxide, reacting with unsaturated bonds of fatty acids. Carboxylic acids containing from 1 to 7 carbon atoms are used, but most often it is peracetic acid, produced in a reversible reaction of acetic acid and hydrogen peroxide in the presence of a mineral acid such as sulfuric acid. The resulting peracid epoxidizes the unsaturated bond of the vegetable oil and the regenerating acetic acid reacts with acetic acid to form a peracid. In known solutions it is introduced in the amount of 0.05 - 0.7 moles for each double bond in one mole of oil. In another solution, the preferred amount of acetic acid is 0.25 - 1.00 moles for each mole of unsaturated bonds. It is also important to select the technological parameters of the process so that the rate of the epoxidation reaction is higher than the reaction of peracid formation. Otherwise, the formed peracid decomposes under the reaction conditions and the efficiency of epoxidation in terms of hydrogen peroxide consumed is low. The smaller the amount of acid used, the lower the reaction rate. In turn, in the case of an excessive amount of acid, the epoxidized product is contaminated with by-products, e.g. hydroxyacetone, which are the result of side reactions of hydrolysis and acylation catalyzed by the mineral acid present in the reaction medium, which lead to the decomposition of the formed epoxy groups, and ultimately this causes a decrease in the selectivity of the transformation to epoxidized oil.

Hydrogen peroxide is usually used in an amount of at least 1.0 mole for each double bond of the ester, with the concentration of the aqueous solution depending on the amount of acid component used. Typically, these are 35-90% solutions, although it is recommended to use solutions containing at least 50% by weight of hydrogen peroxide. Higher concentrations of hydrogen peroxide are used at lower temperatures and with smaller amounts of acetic acid, while more dilute solutions are used at higher temperatures, in combination with larger amounts of acid agent. A small excess of H₂O₂, of the order of 0.1 mole, is usually used, but it can be as much as 1.0 mole.

The most commonly used acid catalysts for the oxidation process are: inorganic acids or their salts, organic sulfonic acids or other organic acids; acid esters; ion exchange resins. An effective acid catalyst should have low solubility in the organic ester phase and be insoluble in the epoxidized ester, which is why inorganic acids such as phosphoric acid and sulfuric acid are most commonly used in amounts from 0.5 to 5% of the total weight of the oxidation mixture. According to the solutions presented in English patent specifications GB739609, GB769127 and GB811852, in the absence of a catalyst or its too small amount, the efficiency of oxidation to epoxy groups decreases significantly. The advantage of conducting the process in the presence of sulfuric acid is a high degree of reacted double bonds and a small amount of by-products. Epoxidation reactions are also catalyzed by basic or permanently neutral salts of metals from groups IA and IIA of the periodic table (acetateof sodium, potassium, lithium, calcium and barium; phosphates of sodium and potassium, bromides and fluorides of sodium and potassium; borates and fluoroborates of sodium and potassium; formates, oxalates, tartrates, citrates and chloroacetates). Hydroxides, oxides, carbonates and bicarbonates of metals from groups I and II of the periodic table can also be used, because they react with acetic acid present in the reaction mixture and form the corresponding acetates. However, salts that oxidize in the reaction medium and salts of heavy metals should not be used, because they catalyze the decomposition of hydrogen peroxide.

The epoxy ring opening reaction is also carried out in the presence of acid catalysts, in this case mainly mineral acids and Lewis acids are used, in the amount of 1 - 4 g per 1 mole of epoxy groups, e.g. phosphoric acid (V), phosphoric acid (III) and phosphoric acid (I), sulfuric acid (VI), p-toluenesulfonic acid. The catalyst can be introduced directly to the reaction mixture or dissolved in the nucleophile, remembering that the introduction of the catalyst causes an increase in the temperature of the reaction mixture. After the reaction process, the catalysts (except phosphoric acid) are removed after the reaction, in order to precisely determine the epoxide value, which is an indicator of the reaction progress. Acids can be removed from the reaction medium by standard methods, e.g. by absorption on solid materials or by neutralization with bases, e.g. NaOH, KOH, Na₂CO₃ or amines, e.g. diethylethanolamine, dimethylethanolamine, ethanolamine, morpholine or ammonia. In the solution presented in the American patent specification No. US4826944, sodium methoxide can also be used for neutralization. Neutralized residues of the acid catalyst remain in the obtained product.

Some of the most commonly used nucleophilic agents for opening rings of epoxidized vegetable oils are water and monohydric alcohols containing in the alkyl chain from 1 to 22 carbon atoms (in the solution known from the American patent specification No. US5380886), but also up to 54 carbon atoms (as presented in another solution known from the American patent specification No. US6057375). These alcohols may contain from 1 to 3 double bonds, a straight or branched hydrocarbon chain, these are usually: methanol, ethanol, 1-propanol, 2-propanol, n-butanol n-pentanol, n-hexanol and 2-ethylhexanol. The second important group consists of polyhydric alcohols with a preferred hydroxyl group content of 2 to 8, e.g. ethylene glycol, diethylene glycol (DEG), polyethylene glycols with molecular weights in the range of 300 - 1500 Da, propane-1,2-diol, propane-1,3-diol, butane-1,4-diol, hexane-1,6-diol, neopentyl glycol, glycerol, sorbitol. According to the solution presented in the American patent specification No. US4825004, it is known that the following were also used: fatty alcohol ether and polyglycerol, fatty alcohol ether containing a linear or branched hydrocarbon chain consisting of 6-22 carbon atoms and from 1 to 3 double bonds; carboxylic acids containing 1 to 8 carbon atoms (preferably 1 - 3), e.g. formic acid, acetic acid, propionic acid; fatty acids containing 6 - 22 carbon atoms, dimers and trimers of fatty acids; ricinoleic acid, hydroxystearic acid, benzoic acid or a mixture of these acids.

It is most advantageous to use vegetable oils containing mixtures of fatty acid groups with a chain length of 6 to 12 carbon atoms. Fatty alcohols containing from 6 to 22 carbon atoms (according to the US patent specification No. US6057375, it is best when they contain from 8 to 18 carbon atoms) can also be used: cyclohexanol, benzyl alcohol, octanol, decanol, lauryl alcohol, myristyl alcohol, cetyl alcohol, stearyl alcohol, oleic alcohol, erucic alcohol or dimers/trimers of diols obtained by hydrogenation of fatty acid esters. In order to ensure the opening of all epoxy rings, nucleophilic agents are used in a stoichiometric amount in relation to the content of epoxy groups in the oil or with a molar excess of 1.05 to 10 times (according to the US patent specification No. US4826944). The molar ratio presented in the solution known from the American patent specification No. US6057375 is usually: 1:2 or 1:3, but extreme ratios are also used, starting from a small excess of the nucleophilic agent, of the order of 1.05 (according to the American patent specification No. US4826944), up to 10:1 (according to the American patent specification No. US5380886). The excess of the nucleophile after the reaction is usually distilled off under reduced pressure.

The disadvantages of existing solutions are:
1. The need to use a solvent, e.g. toluene or methanol, which requires an additional step of its distillation. The production process is taking longer with the steps related to adding, removing and regenerating the solvent. Due to the need to introduce the solvent and the energy used to distill it, the economic aspect of the process deteriorates. Moreover, in the case of insufficient removal of this volatile component, it is transferred into the polyurethane foam together with the NOP polyol (natural oil polyol), causing emissions of volatile organic compounds.
2. There is no effective method of purifying both the oil after the epoxidation reaction and the polyol produced from such oil. As shown below, the purification process has a significant impact on the possibilities of obtaining polyurethane foam with the assumed parameters from NOP polyol.
3. Unfavorable smell properties of the polyol and foams prepared using this polyol. The content of significant amounts of unreacted double bonds in the polyol molecule causes a noticeable characteristic smell of the polyol itself. Preparation of polyurethane foam using such a polyol causes an increase in emissions of smelly compounds due to the heat of reaction of the polyol with isocyanates, as well as due to faster oxidation of double bonds present in the polyol.
4. Lack of stability of NOP polyol over time. Deterioration of the applicational properties of polyol is observed with the passage of time, caused by rancidity of the fatty part of polyol. The term rancidity refers to processes occurring spontaneously in fats during their storage, such as oxidation of fat by autooxidation. During the aging process, peroxides and short aldehydes responsible for the smell and decomposition of the foaming catalyst are released. It was noticed that after a few months the polyol is unfit for use, due to the effect of the lack of foam cross-linking. This phenomenon is related to the consumption of the tin catalyst by peroxides, which are a product of rancidity of polyol containing many unsaturated bonds.
5. Reduced mechanical properties of foams prepared from polyol that was not processed immediately after preparation, even if the deterioration of polyol properties is not yet detectable. It has been observed that even relatively short storage of NOP polyols results in deterioration of mechanical properties of foam, compared to foam prepared using polyol less than a month after ring opening reaction. Properties deteriorate gradually, after several months resulting in the polyol being unsuitable for the preparation of flexible foams with satisfactory mechanical properties.
6. A high content of unreacted nucleophiles or catalyst residues results in reduced stability and durability of polyols, which as the final effect has an adverse effect on foam production, causing degradation of the catalyst in the foaming composition and deterioration of the mechanical properties of the foams, mainly their crosslinking.
7. A large excess of nucleophiles, similarly to the use of solvents, causes the release of large amounts of volatile organic compounds in polyols and the foams produced from them, an effect that is unacceptable to the end users of foams.
8. There are vegetable oils that are naturally characterized by lower double bond contents, which translates into lower iodine values. Examples of such oils are coconut oil and palm oil. Due to the low double bond content, they are more resistant to rancidity. However, the disadvantage of these oils in the context of producing polyols from them is the fact that at room temperature both the oil itself and the epoxides and polyols produced from it are solids, which in practice prevents the use of such a polyol for producing foams.

### The summary of the invention

The polyols that are the subject of the invention are obtained by a two-stage technology: epoxidation and then opening of oxirane (epoxy) rings. In the first stage of such a process, epoxidized vegetable oil is produced from a raw material of natural origin - vegetable oil. In this case, vegetable oil with a reduced content of double bonds, preferably partially hydrogenated, is used. The content of double bonds is selected to the desired hydroxyl value of the polyol in question. The epoxidation process is carried out in an acid environment, usually in the presence of a solvent or without (less often). Due to the desired reduction of volatile organic compounds (which have a significant impact on the smell of the target polyurethane foam), it is advantageous to carry out this reaction in a solvent-free environment. After the first stage reaction is completed, the reaction product, in this case epoxidized oil, is purified. In the second stage, the resulting epoxy bonds are reacted with a nucleophile, which leads to obtaining the actual product - a polyol, which is then used for the production of flexible polyurethane foams. After the second stage reaction is complete, the second purification of the product, in this case the polyol, is performed. Polyols based on natural oils are collectively referred to as NOP (natural oil polyol). The name biopolyol is also encountered.

The measure of the number of double bonds in an organic molecule, for example in oil, is the iodine value (IV). This is the number of grams of halogen, converted to the number of grams of iodine, which under specific conditions undergoes an addition reaction to carbon atoms bonded by a multiple bond, contained in 100 g of the tested substance. Determined in accordance with the EN-ISO 3961:1999 standard. The measure of the number of epoxy bonds in an organic molecule, for example in epoxidized oil, is the epoxide value (EV), expressed in the number of moles of epoxy bonds per 100 g of the tested substance. Determined in accordance with the EN-ISO 3001:1999 standard.

The above features, as well as the disadvantages of the solutions known from the prior art, are listed in Table 1 in comparison with the method according to the invention.

**Table 1**

| | EP4053111A1 | PL206376B1 | PL233221B1 | Chemistry, 2009, 1-Ch, issue 4, pp. 111 - 117 | **The method of the invention** |
|---|---|---|---|---|---|
| Natural raw or refined vegetable oil | Rapeseed oil (RO) with IV according to claims 105 - 120 | P1: Soybean oil (SO) with IV 129 | P1: crude white mustard oil with IV 108, | Palm oil with IV 54 | RO with IV 70 - 90 (reduced from 94 - 120) |
| | P1, 2: IV 110 | P2: RO about IV 112 | | | SO with IV 80 - 115 (reduced from 120 - 139) |
| | P3,7: IV 107 | P3:RO about IV 112 | P2: evening primrose oil lit. IV 147 - 155, | | |
| | P4: IV 109 | | | | P:1,4: RO about IV 86 |
| | P8,9: IV 109 | | P3: Milk thistle oil | | P:2,5: RO about IV 110 |
| | | | | | P:3.6: SO o IV 113 |
| Cleaning after the epoxidation step | Distillation at atmospheric pressure with saturated steam to obtain AV <2. | Separation of the water layer. | Separation of the water layer. | Washing off acids with water. | Wash several times with warm water until acid v. <2. |
| | | Rinse with hot water until the acetic acid is washed out. | Washing with water to wash out acetic acid and catalyst. | Distillation of toluene. | Neutralization (neutralization) with sodium bicarbonate. Drying under vacuum. |
| | Removal of remaining water by centrifugation. | | | | |
| | Neutralization with 30% sodium methoxide (mol 3:1) Rinse with hot water. | Vacuum distillation at 60-70°C. | Removal of remaining water with anhydrous magnesium sulfate. | | Filtration of precipitated salts. |
| | Separation using a pear separator Vacuum drying. | | | | |
| Stage 2 Product (epoxidized oil), EV (mol/100 g) and IV (g/100 g) | 1, 2: EV 0.36 IV 3 | 1: EV 0.216 IV 58.1 | LE 0.273 and IV 42, | White solid, mp 35 - 51°C. | LE in the range of 0.10 - 0.20 and IV < 60 |
| | 3,7: EV 0.35 IV 5 | 2: EV 0.225 IV 46.3 | | | |
| | 4,6: EV 0.18 IV 0 | 3: EV 0.131 IV 61.4 | | 1: EV 0.176 and IV 1.6 | 1,4: EV 0.16 IV 33 |
| | 8,9: EV 0.34 IV 7 | | | 2: EV 0.126 and IV 17.4 | 2,5: EV 0.16 IV 58 |
| | | | | 3: EV 0.069 and IV 33.7 | 3,6: EV 0.16 IV 60 |
| Ring opening stage reaction | Nucleophile: (1 or 2 steps) | Nucleophile: ethylene glycol, catalyzed by (1,2) 85% phosphoric acid or (3) 95% sulfuric acid. | Nucleophile: | Nucleophile: diethylene | Nucleophile: diethylene glycol |
| | 4: glycerin | | thiodiethylene glycol, cat. 96% H₂SO₄ (0.01 - 0.07 mol per epoxy). | glycol to obtain LE=0, | 4: DEG |
| | 6: methanol | | | sulfuric acid catalysis, | 5: DEG |
| | 7: water | | | microwave reactor, | 6: DEG |
| | 9: water | | 4 - 8h, temp. 120°C. | "shortened" reaction time. | |
| Epoxide/Nucleophile Stoichiometry in Step 2 | 1 - 2 moles of nucleophile per mole of epoxy groups in the oil | Stoichiometric quantity (1 mole of nucleophile per 1 mole of epoxy groups of the oil) | Stoichiometric quantity (1 mole of nucleophile per 1 mole of epoxy groups of the oil) | Not specified, probably excess to achieve EV 0. | 0.7 - 1.1 moles of nucleophile per mole of epoxy groups in the oil |
| Method of purifying polyol | Catalyst neutralization. | Neutralization with diethylethanolamine | Neutralization with solid anhydrous calcium chloride. | No information, straw-yellow solids with a melting point of 29 - 46°C were obtained. | Neutralization with sodium bicarbonate Washing with warm water Vacuum drying Filtration of precipitated salts |
| | Rinsing with water until AV 0.4 - 1.2. | | | | |
| | Distillation at atmospheric pressure with saturated steam until AV < 2. | | | | |
| | Vacuum distillation at 80 - 120°C. | | | | |
| Polyol ageing susceptibility | 4.6: moderate | significant due to autoxidation of double bonds, maximum storage time: 1 month | | 1: moderate, maximum storage time: 3 months 2,3: significant, maximum storage time: 1 month | Negligible, maximum storage time: 12 months |
| | 1,2,3,7,8,9: significant due to autoxidation of double bonds, maximum storage time: 1 month | | | | |
| Mechanical properties of elastic foam | OHV values too high (>110) to obtain flexible foam. Deterioration of foam properties during storage of the polyol. | Poor properties of flexible foam due to the use of ethylene glycol. Deterioration of foam properties during storage of polyol due to high IV (>45). | OHV values too high (>290) to obtain flexible foam. | 1, 2: too high OHV (133-166) to obtain flexible foam. | Good, regardless of the polyol storage time. |
| | | | Deterioration of foam properties during storage of polyol due to IV. | The solid form makes it practically impossible to apply to flexible foams. | |
| | Deterioration of properties due to excess nucleophile. | | | | |
| The smell of foam | Characteristic | Characteristic | Not applicable, no possibility of producing flexible foam. Smell of the polyol itself deteriorated due to the use of organosulfur nucleophile. | Not applicable, no possibility of producing flexible foam. | Imperceptible or almost imperceptible |

The method according to the invention solves the technical problem consisting in the impossibility of obtaining NOP polyols (i.e. polyols from natural, vegetable oils) which are simultaneously characterized by a hydroxyl value not higher than 120 mg KOH/g, preferably 110 mg KOH/g, and therefore find use in the production of flexible foams, and have favorable smell properties.

The subject of the invention is therefore a method for producing a natural polyol based on natural vegetable oil (preferably rapeseed or soybean) with a reduced content of unsaturated bonds and its subsequent use for producing flexible polyurethane foams characterized by a low (imperceptible) smell and similar mechanical properties compared to foams obtained on the basis of petrochemical polyether polyols (i.e. obtained on the basis of raw materials originating from crude oil processing). Foams obtained on NOP polyols are characterized by technical parameters similar to commonly used products of petrochemical origin, and they are superior to these polyols in terms of flame retardant properties of foams.

During the research, without wishing to be bound by any theory, it unexpectedly turned out that double bonds in vegetable oils are not equal in terms of deterioration of the properties of the final polyol. In particular, partial hydrogenation of the oil combined with a double purification process, first of the epoxidized oil and then of the polyol, is sufficient to eliminate the unfavorable smell of the polyol and the foam prepared from it, as well as the effect of deterioration of the properties of the polyol during storage. Partial hydrogenation of raw or refined oil is aimed at reducing the most reactive double bonds, which are responsible for the smell in the polyol. In the epoxidation stage, a certain part of the double bonds remaining after partial hydrogenation is converted into epoxy bonds, which are then opened in the next stage. It is likely that the double bonds remaining after these two stages are the least reactive, which is why they are least subject to oxidation in the rancidity process during storage. At the same time, partial hydrogenation does not cause the oil to harden into a solid fat, resulting in the solidification of both the starting oil, epoxide and the final polyol. All polyols produced by the method according to the invention are liquid at room temperature. Therefore, despite the iodine value of the polyol prepared by the method according to the invention being in the range of 30 - 60 g/100 g, the aforementioned disadvantageous effects known from the prior art solutions are not observed.

During the conducted research it also unexpectedly turned out that the selection of the method of purification of the polyol is of key importance in obtaining a stable polyol useful for obtaining flexible polyurethane foams, in combination with the use of vegetable oil with a reduced content of double bonds. Various methods of purification of NOP polyols are known in the literature, but most often the inventors do not pay much attention to the applied purification method. From the experience of the inventors of the present invention it results, however, that, in combination with the use of partially hydrogenated vegetable oil, the selection of the appropriate purification method is key to maintaining the advantageous properties of the polyol during storage.

Thus, by the process according to the invention, polyols for use in foams, particularly flexible foams, can be obtained with the following desired properties: acid value below 1 mg KOH/g, preferably below 0.1 mg KOH/g, hydroxyl value below 120 mg KOH/g, viscosity at 25°C below 3000 mPas, molecular weight in the range of 3000 - 5000 Da. They also have a low level of volatile organic impurities (VOC), below 250 ppm, and a low concentration of free nucleophile - below 2 wt. %, preferably below 0.6 wt. %.

**Table 2 - comparison of polyol purification methods.**

| | **Method A** | **Method B** | **Method C** | **Method D (according to the invention)** |
|---|---|---|---|---|
| Sub-stage 1 | Neutralization | Neutralization | Wash | Neutralization |
| Sub-stage 2 | Drying | Drying | Drying | Wash |
| Sub-stage 3 | Filtering | Filtering | - | Drying |
| Sub-stage 4 | - | Wash | - | Filtering |
| Sub-stage 5 | - | Drying | - | - |
| Technical effect | | | | unnoticeable/negligible smell, good foam properties |

The unique combination of the steps of carrying out the method according to the present invention allows to obtain the final effect - a polyol and foam with an imperceptible smell, which has parameters comparable to foams obtained from polyols of petrochemical origin existing on the market. The use of oil with a reduced content of double bonds by at least 10% mol. relative to crude or refined natural oil (i.e. obtained by methods known to a person skilled in the art and not subjected to hydrogenation), wherein the content of double bonds is determined by means of the iodine value (IV), i.e. preferably in the case of rapeseed oil with an iodine value, determined in accordance with EN -ISO 3961:1999, below 100 g/100 g, and in the case of soybean oil with an iodine value below 120 g/100 g, results in obtaining in the first stage of the process an epoxidized oil, which, when used subsequently in the next stage of the process, consisting in the complete opening of oxirane rings and then appropriate purification, results in obtaining a polyol practically free of epoxy bonds, suitable for obtaining flexible foams with reduced smell. However, the use of a smaller amount of nucleophile, such as diethylene glycol, and the application of a purification method in the next stage allows for an additional reduction in the content of volatile organic compounds (TVOC) in the foam and improves its mechanical properties.

In a first aspect, the invention relates to a method for obtaining a polyol from a vegetable oil without using a solvent, comprising the following steps:
a) providing a mixture containing:
   a1) a vegetable oil with a double bond content reduced by 10-40 mol%, preferably 15-35 mol%, determined using the iodine value in accordance with EN-ISO 3961:1999, relative to the crude vegetable oil, and
   a2) a mixture of acids: 80% acetic acid and 95-98% sulfuric acid (VI) in weight proportions of 6:1,
b) epoxidation of the mixture from step a) by adding hydrogen peroxide for 90 to 120 minutes at a constant reaction temperature of 50 - 65°C; then stirring is continued for a period of 2 to 6 hours after the end of the hydrogen peroxide addition,
c) purification of the epoxidized oil obtained in step b) comprising the substeps:
   c1) separation of the organic layer containing epoxidized oil,
   c2) washing several times with water at a temperature of 50 - 65°C until the acid value is below 2 mg KOH/g, determined in accordance with,
   c3) neutralization with a basic salt in a stoichiometric amount relative to the acid value,
   c4) drying the organic layer by heating to a temperature in the range of 80 - 90°C under pressure below manometric zero while stripping with the nonreactive gas to a water content below 0.1% by weight in the polyol,
   c5) filtration of precipitated salts,
d) contacting the epoxidized oil obtained from step c) in the presence of an acid catalyst with dialkyl glycol used in an amount of 0.7 - 1.1 mol. relative to the content of epoxy groups in the epoxidized oil,
e) purifying the polyol obtained from step d) under the conditions described in substeps c1) to c5).

Preferably, in the method the vegetable oil is selected from rapeseed oil having an iodine value, determined in accordance with EN-ISO 3961:1999, of less than 100 g/100 g, preferably in the range of 70 - 90 g/100 g, or soybean oil having an iodine value of less than 120 g/100 g, preferably 80 - 115 g/100 g, or a mixture thereof,

Preferably, in the method, acetic acid is used in a ratio of 0.15 to 2.1 mol. to the content of unsaturated bonds in the vegetable oil, and sulfuric acid is used in an amount of 2.0 - 2.5 mol.% relative to the total mass of the organic acid and hydrogen peroxide.

Preferably, the method uses 35% hydrogen peroxide in a molar ratio to the content of unsaturated bonds in the vegetable oil of from 0.5 to 0.8.

Preferably, the basic salt in step c3) is a salt of a strong base and a weak acid in an amount of 2:1 mol. with respect to the amount of acid catalyst used, preferably sodium bicarbonate.

Preferably, 98% sulfuric acid is used as the acid catalyst in step d) in an amount of from 0.1 to 0.2% by weight based on the weight of the epoxidized oil, preferably 1 g of acid per 1 mole of epoxy groups in the epoxidized oil.

Preferably the dialkyl glycol is diethylene glycol.

In a further aspect, the invention relates to a natural polyol obtained by the method as described above.

Preferably, the polyol has a free diethylene glycol content of less than 1% by weight, preferably less than 0.6% by weight, and a volatile organic compound content of less than 350 ppm, determined by heating a sample of the polyol in a container and then analyzing the vapors based on VDA 277.

In a further aspect, the invention relates to a method of producing polyurethane foam comprising mixing:
- a natural polyol as defined above in an amount of less than 60 parts by weight, preferably in the range of 5 - 30 parts by weight, based on 100 parts by weight of polyols in the mixture,
- a polyether polyol, being a copolymer of ethylene oxide and propylene oxide with an average molecular weight of 3000 - 5000 Da, preferably in an amount of 40 - 95 parts by weight per 100 parts by weight of polyols in the mixture,
- distilled water, preferably in an amount of 3.0 - 5.0 parts by weight per 100 parts by weight of polyols in the mixture,
- a foaming amine, preferably a solution of 70% bis(N,N-dimethylaminoethyl)ether in dipropylene glycol in the amount of 0.04 - 0.06 parts by weight per 100 parts by weight of polyols in the mixture,
- gelling amine, preferably a solution of 33% triethylenediamine in propylene glycol in the amount of 0.14 - 0.16 parts by weight per 100 parts by weight of polyols in the mixture
- a surfactant, preferably an organosilicon compound in an amount of 0.9 - 1.1 parts by weight per 100 parts by weight of polyols in the mixture,
- a catalyst, preferably tin (II) octoate in an amount of 0.1 - 0.3 parts by weight per 100 parts by weight of polyols in the mixture,
and then contacting the resulting mixture with 51 - 53 parts by weight of isocyanate based on 100 parts by weight of polyols in the mixture, preferably tolylene diisocyanate (TDI).

In a further aspect, the invention relates to a polyurethane foam obtained by the method as defined above.

Preferably the foam is flexible block foam.

The foam preferably has the following parameters: density in the range of 20 - 60 kg/m³, determined in accordance with ISO 845, hardness in the range of 1 - 7 kPa, determined in accordance with ISO3386-1, resilience in the range of above 35%, determined by the Ball Rebound method in accordance with ISO 8307, compression set 50% in the range of below 7%, determined in accordance with ISO 1856, method A, elongation at break in the range of above 50%, determined in accordance with PN-EN ISO 1798, tensile strength in the range of above 40 kPa, determined in accordance with PN-EN ISO 1798.

Thus, the method according to the invention makes it possible to obtain a natural polyol and use it to obtain a foam with the following advantages.
1. Extended stability and durability of the product - polyol and foam. The effect of lack of foam cross-linking, related to the consumption of the tin catalyst by peroxides, which are a product of aging over time of the polyol containing many reactive unsaturated bonds, has been eliminated.
2. Eliminated unpleasant smell in the final product - natural polyol (NOP) and flexible polyurethane foam. The smell of the foam and polyol is caused by double bonds, epoxy rings, and decomposition products of these bonds, such as short aldehydes, which are decomposition products of peroxides. The heat released during the reaction of isocyanates with polyol during foam preparation accelerates unfavorable ageing reactions, as a result of which unfavorable smell effects in foams are noticeable even after preparation of foams from freshly prepared NOP polyols.
3. Similar mechanical properties of flexible polyurethane foam using up to 50 parts by weight, preferably up to 30 parts by weight of a natural polyol obtained according to the described method, in the foam composition as compared to petrochemical polyols (not produced on the basis of natural oil raw materials).
4. Positive effect on the non-flammable properties of flexible polyurethane foam (reduction of the liquid flame retardant level by at least 20% in the foam composition in the MVSS302 flammability test).

### Detailed description of the invention

In a first aspect, the invention relates to a method of obtaining a polyol from a vegetable oil without using a solvent, comprising the following steps:
a) providing a mixture containing:
   a1) a vegetable oil with a double bond content reduced by 10-40 mol %, preferably 15-35 mol %, determined using the iodine value in accordance with EN-ISO 3961:1999, relative to the crude vegetable oil, and
   a2) a mixture of acids: 80% acetic acid and 95-98 % sulfuric acid (VI) in weight proportions of 6:1,
b) epoxidation of the mixture from step a) by adding hydrogen peroxide for 90 to 120 minutes at a constant reaction temperature of 50 - 65°C; then stirring is continued for a period of 2 to 6 hours after the end of the hydrogen peroxide addition,
c) purification of the epoxidized oil obtained in step b) comprising the substeps:
   c1) separation of the organic layer containing epoxidized oil,
   c2) washing several times with water at a temperature of 50 - 65°C until the acid value is below 2 mg KOH/g, determined in accordance with,
   c3) neutralization with a basic salt in a stoichiometric amount relative to the acid value,
   c4) drying the organic layer by heating to a temperature in the range of 80 - 90°C under pressure below manometric zero while stripping with the nonreactive (inert) gas to a water content below 0.1% by weight in the polyol,
   c5) filtration of precipitated salts,
d) contacting the epoxidized oil obtained from step c) in the presence of an acid catalyst with dialkyl glycol used in an amount of 0.7 - 1.1 mol. relative to the content of epoxy groups in the epoxidized oil,
e) purifying the polyol obtained from step d) under the conditions described in substeps c1) to c5).

Preferably, any oil of vegetable origin having a reduced content of double bonds, preferably by (partial) hydrogenation, can be used in the method. Preferably, the vegetable oil is selected from rapeseed oil, soybean oil, castor oil, sunflower oil, palm oil, white mustard oil, evening primrose oil, milk thistle oil. Even more preferably, the vegetable oil is selected from rapeseed oil having an iodine value, determined in accordance with EN-ISO 3961:1999, of less than 100 g/100 g, most preferably in the range of 70-90 g /100 g, or soybean oil having an iodine value of less than 120 g/100 g, most preferably 80-115 g /100 g, or a mixture thereof.

Preferably, in the method, acetic acid is used in a ratio of 0.15 to 2.1 mol. to the content of unsaturated bonds in the vegetable oil, and sulfuric acid is used in an amount of 2.0 - 2.5 mol.% relative to the total mass of the organic acid and hydrogen peroxide.

Preferably, the method uses 35% hydrogen peroxide in a molar ratio to the content of unsaturated bonds in the vegetable oil of from 0.5 to 0.8.

Preferably, the basic salt in step c3) is a salt of a strong base and a weak acid in an amount of 2:1 mol. with respect to the amount of acid catalyst used, preferably monovalent metal hydrogen carbonates, more preferably sodium hydrogen carbonate.

Preferably, 98% sulfuric acid is used as the acid catalyst in step d) in an amount of from 0.1 to 0.2% by weight based on the weight of the epoxidized oil, preferably 1 g of acid per 1 mole of epoxy groups in the epoxidized oil.

Preferably, the dialkyl glycol may be any dialkyl glycol known in the art, more preferably diethylene glycol.

In a further aspect, the invention relates to a natural polyol obtained by the method as described above.

Preferably, the polyol has a free diethylene glycol content of less than 1% by weight, preferably less than 0.6% by weight, and a volatile organic compound content of less than 350 ppm, determined by heating a sample of the polyol in a container and then analysing the vapours based on VDA 277.

In a further aspect, the invention is a method of producing polyurethane foam comprising mixing:
- a natural polyol as defined above in an amount of less than 60 parts by weight, preferably in the range of 5 - 30 parts by weight, based on 100 parts by weight of polyols in the mixture,
- a polyether polyol, being a copolymer of ethylene oxide and propylene oxide with an average molecular weight of 3000 - 5000 Da, preferably in an amount of 40 - 95 parts by weight per 100 parts by weight of polyols in the mixture,
- distilled water, preferably in an amount of 3.0 - 5.0 parts by weight per 100 parts by weight of polyols in the mixture,
- a foaming amine, preferably a solution of 70% bis(N,N-dimethylaminoethyl)ether in dipropylene glycol in the amount of 0.04 - 0.06 parts by weight per 100 parts by weight of polyols in the mixture,
- gelling amine, preferably a solution of 33% triethylenediamine in propylene glycol in the amount of 0.14 - 0.16 parts by weight per 100 parts by weight of polyols in the mixture
- a surfactant, preferably an organosilicon compound in an amount of 0.9 - 1.1 parts by weight per 100 parts by weight of polyols in the mixture,
- a catalyst, preferably tin (II) octoate in an amount of 0.1 - 0.3 parts by weight per 100 parts by weight of polyols in the mixture,
and then contacting the resulting mixture with 51 - 53 parts by weight of isocyanate based on 100 parts by weight of polyols in the mixture, preferably tolylene diisocyanate (TDI).

In a further aspect, the invention relates to a polyurethane foam obtained by the method as defined above.

Preferably the foam is a flexible block foam.

The foam preferably has the following parameters: density in the range of 20 - 60 kg/m³, determined in accordance with ISO 845, hardness in the range of 1 - 7 kPa, determined in accordance with ISO3386-1, resilience in the range of above 35%, determined by the Ball Rebound method in accordance with ISO 8307, compression set 50% in the range of below 7%, determined in accordance with ISO 1856, method A, elongation at break in the range of above 50%, determined in accordance with PN-EN ISO 1798, tensile strength in the range of above 40 kPa, determined in accordance with PN-EN ISO 1798.

The method of producing a polyol from a vegetable oil according to the invention is characterized by that in the first stage, a process of obtaining an epoxidized vegetable oil with a reduced content of double bonds is carried out, consisting in the reaction of a partially hydrogenated vegetable oil with an organic peracid formed "in situ" during mixing of an organic acid with hydrogen peroxide in the presence of an mineral acid catalyst, without the participation of an organic solvent. Hydrogen peroxide is introduced for 90 to 120 minutes so as to maintain a constant reaction temperature of 50 - 65°C, during intensive mixing, then mixing is continued for 2 to 6 hours from the end of the introduction of hydrogen peroxide, and then the post-reaction product is subjected to separation into an organic layer and an aqueous layer, after which the organic layer is washed several times with water until the acid value in the organic layer is below 2.0 mg KOH/g, and after each washing the organic layer is separated from the waste aqueous layer. In the next unit operation, the organic layer is neutralized, then dried under reduced pressure (below manometric zero) while stripping with nitrogen and filtered to separate precipitated salts. In the second stage, the oxidized oil with reduced content of double bonds is reacted with a nucleophile in the presence of an acid catalyst in an amount close to stoichiometric in relation to the content of epoxy groups in the oil. The reaction is carried out at a temperature of 100 to 120°C for a period of 3 to 5 hours, the post-reaction product is then cooled to 80°C and neutralized and washed with water, the organic layer is separated from the water layer, then the organic layer is dried under reduced pressure (below manometric zero) while stripping with nitrogen and filtered to separate precipitated salts. The polyol obtained in this way is then used for the production of flexible polyurethane foam.

### Methods

The hydroxyl value was determined in accordance with ASTM D4274-16 method D.

The iodine value was determined in accordance with EN-ISO 3961:1999.

The epoxide value was determined according to EN-ISO 3001:1999.

The acid value was determined according to ASTM D7253-16.

The viscosity was determined in accordance with ASTM D4878-15, method A.

The molecular weight was determined by gel permeation chromatography (GPC) based on polystyrene molecular weight standards.

The VOC pollutant emission level was determined in accordance with VDA 277.

The density of the foams was determined according to the ISO 845 method.

The hardness of the foams was determined according to ISO3386-1.

The elasticity/resilience of the foams was determined using the Ball Rebound method, according to ISO 8307.

The compression set was determined in accordance with ISO 1856 method A.

Elongation at break was determined in accordance with PN-EN ISO 1798.

The tensile strength was determined in accordance with PN-EN ISO 1798.

### Examples

### Epoxy (epoxidized oil)

### Example 1

500 g of partially hydrogenated rapeseed oil (iodine value 86 g/100 g) and a mixture of acids: 26.0 g of 80% acetic acid and 4.0 g of 95% sulfuric acid (VI) were introduced into a 1000 ml glass reactor equipped with a reflux condenser, thermometer and stirrer. The reactor contents were heated to 45°C and then 125.0 g of 35% wt. H₂O₂ was introduced over 1.5 hours. After adding the entire amount of H₂O₂, the reaction was carried out for 4 h at a temperature between 60 and 65°C. Then the lower aqueous layer was separated and the organic layer was washed several times with water at a temperature in the range of 60 and 80°C until the acid value was below 2.0 mg KOH/g. Then, sodium bicarbonate was added for neutralization in an amount corresponding to the acid value of the organic layer after washing. The neutralized product was dried at a temperature in the range of 80 - 90°C under reduced pressure for 4 hours, while stripping with nitrogen, until the water content was below 0.06% by weight. Then, the precipitated salts were filtered on a sintered filter funnel. The reaction product is a light yellow, clear epoxidized rapeseed oil with an iodine value of 33.0 g/100g, an epoxide value of 0.16 mol/100g and an acid value of <0.1 mg KOH/g. The product is liquid at room temperature and has a mild smell. All parameters are maintained even after 6 months of epoxy storage.

### Example 2 (comparative)

The procedure was the same as in Example 1, with the difference that 500 g of non-hydrogenated rapeseed oil with an iodine value of 110 and the following amounts of other reagents were used: 26.0 g of 80% acetic acid, 4.0 g of 95% sulfuric acid (VI) and 125.0 g of 35% hydrogen peroxide. A light yellow, clear epoxidized rapeseed oil was obtained with an iodine value of 58.0 g/100 g, an epoxide value of 0.17 mol/100 g and an acid value of <0.1 mg KOH/g. The product is liquid at room temperature and has a mild smell. After 3 months of storage, the smell of the oil is sensorially noticeably worse, the most common descriptor of the assessors is "mouse smell".

### Example 3

The procedure was the same as in Example 1, with the difference that 500 g of partially hydrogenated soybean oil with an iodine value of 113 g/100 g and the following amounts of other reactants were used: 26.0 g of 80% acetic acid, 4.0 g of 95% sulfuric acid (VI) and 125.0 g of 35% hydrogen peroxide. A light yellow, clear epoxidized soybean oil with an iodine value of 60.0 g/100 g and an epoxide value of 0.17 mol/100 g was obtained. The product is liquid at room temperature and has a mild smell. All parameters are maintained even after 6 months of epoxide storage.

### Polyol

### Comparative examples 4A-C and example 4D according to the invention

Into a 2000 ml glass reactor equipped with a thermometer and a stirrer were introduced 1000 g of epoxidized partially hydrogenated rapeseed oil with an iodine value of 33 g/100 g and an epoxide value of 0.16 mol/100 g, obtained in Example 1, 192.0 g of diethylene glycol and 1.5 g of 95% sulfuric acid (VI) dissolved in glycol. The reaction was carried out at a temperature in the range of 110 - 120°C for 5 hours (until all epoxy bonds present in the oil were opened) under a nitrogen blanket. After the reaction was completed, the sulfuric acid was neutralized with sodium bicarbonate added in a stoichiometric amount, and then the product was washed with water at a temperature in the range of 60 - 80°C. The neutralized product was dried at a temperature in the range of 80 - 90°C under reduced pressure with simultaneous stripping with nitrogen, until the water content was below 0.08%. Then the precipitated salts were filtered on a sintered funnel. The reaction product is a yellow liquid with a mild smell, with an iodine value of 33 g/100 g, an epoxide value of 0, an acid value of <0.1 mg KOH/g, and a hydroxyl value of 110 mg KOH/g. All parameters are maintained even after 6 months of polyol storage.

For comparative purposes, the synthesis was repeated except that instead of using the purification method according to (method D of Table 2), comparative methods A, B and C were used. The results are summarized in Table 3.

**Table 3**

| | **Method A** | **Method B** | **Method C** | **Method D** |
|---|---|---|---|---|
| Acid value, mg KOH/g | 2.90 | 3.78 | 4.35 | <0.1 |
| Average molecular weight, Da | 3050 | 3120 | 3380 | 2830 |
| VOC, ppm, eq. toluene | 353 | 140 | 198 | 95 |
| Free DEG content, % wt. | 2.55 | 0.23 | 0.1 | 0.1 |
| Volatile organic compounds in polyol, mg/kg | 353 | 140 | 198 | 95 |
| Flexible foam with a density of 24 kg/m³ prepared using polyol | Bad structure, closed cells | Non-crosslinked foam, "cotton candy" effect | Non-crosslinked foam, "cotton candy" effect | Correct foam with a fine, open structure |

As can be seen from Table 3 above, the purification method (sequence of steps) has a significant effect on the properties of the polyol, in particular on the content of free diethylene glycol (DEG) and volatile organic compounds (VOC), and the foam obtained from it. Free glycol means glycol (nucleophile) not chemically bound to the polyol molecules, remaining after the ring opening reaction.

### Example 5 (comparative)

The procedure was the same as in Example 4, with the difference that epoxidized rapeseed oil with an iodine value of 58 g/100 g and an epoxide value of 0.17 obtained in Comparative Example 2 was used, as well as the following amounts of the remaining reagents: 130.0 g of diethylene glycol and 1.5 g of 95% sulfuric acid (VI). The obtained polyol was a clear, yellow liquid with a mild smell, with an iodine value of 58.0 g/100 g, an epoxide value of 0, an acid value of <0.1 mg KOH/g and a hydroxyl value of 112 mg KOH/g. After 3 months of storage, the smell of the oil is sensorially clearly worse, the most common descriptors of the assessors are "smell of old rags" and "smell of mice".

### Example 6

Proceeding as in Example 4 and using epoxidized soybean oil (IV = 60 and EV = 0.17) obtained in Example 3 and the following amounts of other reactants: 130.0 g of diethylene glycol and 1.5 g of 95% sulfuric acid (VI). A polyol was obtained in the form of a clear, light yellow liquid with a mild smell, with an iodine value of 60 g/100 g, an epoxide value of 0, an acid value of 0.2 mg KOH/g and a hydroxyl value of 110 mg KOH/g. All parameters are maintained even after 6 months of storage of the polyol.

The properties of the obtained polyols are compared in Table 4.

**Table 4**

| **Properties of polyols** | **P4D** | **P5 comp.** | **P6** |
|---|---|---|---|
| Epoxidized oil according to the Example | P1 | P2 comp. | P3 |
| Iodine value, g/100g | 33 | 58 | 60 |
| Epoxide value, mol/100g | 0 | 0 | 0 |
| Hydroxyl value, mg KOH/g | 110 | 112 | 110 |
| Acid value, mg KOH/g | <0.1 | <0.1 | 0.2 |
| Sensory evaluation* of the smell after 6 months of storage | Advantageous: "mild or no smell" | Unfavorable: "the smell of old rags, the smell of mice" | Advantageous: "mild smell" |

| | | | |
|---|---|---|---|
| * Sensory evaluation was based on an individual assessment by a panelist consisting of 12 persons skilled in the art. The study was conducted as a blind test. | | | |

### Foam

Without wishing to be bound by theory, it has been determined that, most likely due to steric hindrance, only about 50% of the OH groups in the NOP polyols are accessible to the NCO groups of the isocyanates during the foaming process; to maintain the flexibility of the foam, correspondingly half as much isocyanate must be used. Experimentally, it has been determined that the optimum foam properties are obtained with not more than 60% by weight, and preferably not more than 30% by weight, of the NOP polyols according to the invention.

### Example 7

Polyols and additives according to the following composition were mixed together in a 1-liter vessel for 30 s, at a mixer speed of 3500 rpm. Then isocyanate was added and mixed for another 8 s. The whole was poured into a paper mold. The obtained flexible free-rise polyurethane foams were conditioned for 1 h at 100°C and then for 47 h at ambient temperature (20 - 24°C).

Composition:
- 20 g Polyol A - natural polyol NOP obtained according to Example 4, with an iodine value of 33 g/100 g and a hydroxyl value of 110 - 120 mg KOH/g.
- 80 g Polyol B - "polyether polyol", a copolymer of ethylene oxide and propylene oxide with an average molecular weight of 3600 Da
- 4.0 g Water - distilled water
- 0.05 g Amine 1 - foaming amine 70% solution of Bis (N,N-dimethylaminoethyl) ether in dipropylene glycol Dabco BL11
- 0.15 g Amine 2 - gelling amine, 33% solution of triethylenediamine in propylene glycol Dabco 33LV
- 1.0 g Surfactant - organosilicon compound Tegostab BF2370,
- 0.2 g tin (II) octanoate Kosmos 29
- 52 g TDI - toluene diisocyanate Lupranat T80.

### Example 8 (comparative)

The procedure was as in Example 7, with the difference that the NOP polyol obtained according to Example 5 was used as Polyol A, to obtain polyurethane foam.

### Example 9

The procedure was as in Example 7, with the difference that the NOP polyol obtained according to Example 6 was used as Polyol A, obtaining polyurethane foam.

### Examples 10, 11 (comparative) and 12

The foaming procedure of Examples 7, 8 (comparative) and 9 was repeated with the difference that before starting to produce polyurethane foam, the polyols obtained in Examples 4, 5 (comparative) and 6, respectively, were stored in closed packages at ambient temperature for 6 months. The foams produced by the method according to the invention showed parameters similar to those of the foams produced directly after the preparation of the polyol. However, in the comparative example, it was not possible to obtain foam with the assumed parameters. The ageing changes occurring in the comparative polyol from Example 5 caused problems with cross-linking under foaming conditions, which prevented application.

### Example 13 (comparative)

The procedure was the same as in Example 7, with the difference that Polyol A was not used in the composition at all, replacing it completely with polyol B, thus obtaining polyurethane foam.

Table 5 presents the measurement results of selected properties of polyurethane foams obtained according to Examples 7 - 12.

**Table 5**

| **Foam properties** | **P.7** | **P. 8 comp.** | **P.9** | **P. 10** | **P. 11 comp.** | **P. 12** | **P. 13 comp.** |
|---|---|---|---|---|---|---|---|
| **Density [kg/m ³]** | 29.3 | 30.0 | 30.8 | 29.0 | Non-crosslinked foam, "cotton candy" effect, no possibility to assess mechanical properties | 29.6 | 30.0 |
| **Hardness CV40 [kPa]** | 2.5 | 3.2 | 3.2 | 2.6 | | 2.9 | 3.2 |
| **Comfort factor** | 2.4 | 2.4 | 3 | 2.5 | | 2.8 | 2.5 |
| **Air flow [dm ³/s]** | 3.0 | 2.2 | 0.9 | 2.8 | | 1.6 | 2.9 |
| **Resilience [%]** | 44 | 45 | 41 | 46 | | 43 | 45 |
| **Compression set, dry 50%** | 8.6 | 9.0 | 8.3 | 8.9 | | 8.1 | 8.1 |
| **Tensile strength [kPa]** | 95.3 | 99 | 92 | 98.0 | | 90 | 92 |
| **Relative elongation at break [%]** | 157 | 153 | 151 | 160 | | 148 | 155 |
| **The smell of foam** | Imperc eptible | Characteri stic | Imperceptible | | Unaccepta ble | Imperceptible | |

The influence of the nucleophile on the foam properties is presented in Table 6. The polyols were obtained according to the method described in Example 1, and the foams - according to the method described in Example 7 with the differences indicated in Table 6.

**Table 6**

| | **Required ranges** | **P. 14 comp.** | **P. 15 comp.** | **P. 16 comp.** | **P. 17 comp.** |
|---|---|---|---|---|---|
| Acid value | <1mgKOH/g | 2.12 | 1.43 | 1.67 | 1.54 |
| Hydroxyl value | *110 - 120 mg KOH*/*g* | 76.02 | 113.74 | 69.2 | 73.05 |
| Molecular weight [Da] | *3000 - 5000* | 5770 | 3300 | 11300 | 4980 |
| Nucleophile [% mole] | | 0.70 | 1.00 | 1.00 | 1.00 |
| TVOC [ppm] eq. toluene | | 151 | 709 | 2023 | 4024 |
| Nucleophile | | Ethylene glycol | Ethylene glycol | Isopropanol | Isobutanol |
| Foam | | "cotton candy" | | parameters outside the required ranges | |

Table 6 shows that other nucleophiles are not suitable for obtaining polyols with the given parameters because the foams produced from them have defects.

## Claims

1. A method for obtaining a polyol from a vegetable oil without using a solvent, comprising the following steps:
a) providing a mixture containing:
a1) a vegetable oil with a double bond content reduced by 10-40 mol %, preferably 15-35 mol %, determined using the iodine value in accordance with EN-ISO 3961:1999, relative to the crude vegetable oil, and
a2) a mixture of acids: 80% acetic acid and 95-98 % sulfuric acid (VI) in weight proportions of 6:1,
b) epoxidation of the mixture from step a) by adding hydrogen peroxide for 90 to 120 minutes at a constant reaction temperature of 50 - 65°C; then stirring is continued for a period of 2 to 6 hours after the end of the hydrogen peroxide addition,
c) purification of an epoxidized oil obtained in step b) comprising the following substeps:
c1) separation of an organic layer containing the epoxidized oil,
c2) washing several times with water at a temperature of 50 - 65°C until the acid value is below 2 mg KOH/g, determined in accordance with ASTM D7253-16,
c3) neutralization with a basic salt in a stoichiometric amount relative to the acid value,
c4) drying the organic layer by heating to a temperature in the range of 80 - 90°C under pressure below manometric zero while stripping with the nonreactive gas to a water content below 0.1% by weight in a polyol,
c5) filtration of precipitated salts,
d) contacting the epoxidized oil obtained from step c) in the presence of an acid catalyst with a dialkyl glycol used in an amount of 0.7 - 1.1 mol. relative to the content of epoxy groups in the epoxidized oil,
e) purifying a polyol obtained from step d) under the same conditions as in substeps c1) to c5).

2. The method according to claim 1, wherein the vegetable oil is selected from a rapeseed oil having an iodine value, determined in accordance with EN-ISO 3961:1999, of less than 100 g/100 g, preferably in the range of 70 - 90 g/100 g, or a soybean oil having an iodine value of less than 120 g/100 g, preferably 80 - 115 g/100 g, or a mixture thereof.

3. The method according to claim 1 or 2, wherein acetic acid is used in a ratio of 0.15 to 2.1 mol. to the content of unsaturated bonds in the vegetable oil, and sulfuric acid is used in an amount of 2.0 - 2.5 mol.% relative to the total weight of the organic acid and hydrogen peroxide.

4. The method according to any one of claims 1-3, wherein 35% hydrogen peroxide is used in a molar ratio to the content of unsaturated bonds in the vegetable oil of from 0.5 to 0.8.

5. The method according to any one of claims 1-4, wherein the basic salt in step c3) is a salt of a strong base and a weak acid in an amount of 2:1 mol. with respect to the amount of the acid catalyst used, preferably sodium bicarbonate.

6. The method according to any one of claims 1 to 5, wherein 98% sulfuric acid is used as the acid catalyst in step d) in an amount of 0.1 to 0.2% by weight based on the weight of the epoxidized oil, preferably 1 g of acid per 1 mole of epoxy groups in the epoxidized oil.

7. The method of any one of claims 1 to 6, wherein the dialkyl glycol is diethylene glycol.

8. A natural polyol obtained by the method of any one of claims 1 to 8.

9. The polyol of claim 8, wherein the polyol has a free diethylene glycol content of less than 1% by weight, preferably less than 0.6% by weight, and a volatile organic compound content of less than 350 ppm, determined by heating a sample of the polyol in a container and then analysing the vapours based on VDA 277.

10. A method of producing polyurethane foam comprising mixing:
- a natural polyol according to claim 8-9 in an amount of less than 60 parts by weight, preferably in the range of 5 - 30 parts by weight, based on 100 parts by weight of polyols in the mixture,
- a polyether polyol, being a copolymer of ethylene oxide and propylene oxide with an average molecular weight of 3000 - 5000 Da, preferably in an amount of 40 - 95 parts by weight per 100 parts by weight of polyols in the mixture,
- distilled water, preferably in an amount of 3.0 - 5.0 parts by weight per 100 parts by weight of polyols in the mixture,
- a foaming amine, preferably a solution of 70% bis(N,N-dimethylaminoethyl)ether in dipropylene glycol, in the amount of 0.04 - 0.06 parts by weight per 100 parts by weight of polyols in the mixture,
- a gelling amine, preferably a solution of 33% triethylenediamine in propylene glycol in the amount of 0.14 - 0.16 parts by weight per 100 parts by weight of polyols in the mixture
- a surfactant, preferably an organosilicon compound in the amount of 0.9 - 1.1 parts by weight per 100 parts by weight of polyols in the mixture,
- a catalyst, preferably tin (II) octoate in an amount of 0.1 - 0.3 parts by weight per 100 parts by weight of polyols in the mixture,
and then contacting the resulting mixture with 51 - 53 parts by weight of an isocyanate based on 100 parts by weight of polyols in the mixture, preferably toluene diisocyanate (TDI).

11. A polyurethane foam produced by the method as defined in claim 10.

12. The foam of claim 11, wherein the foam is flexible block foam.

13. The foam according to any one of claims 14 or 15, wherein the foam has the following parameters: density in the range of 20 - 60 kg/m³, determined in accordance with ISO 845, hardness in the range of 1 - 7 kPa, determined in accordance with ISO3386-1, resilience in the range of above 35%, determined by the Ball Rebound method in accordance with ISO 8307, compression set 50% in the range of below 7%, determined in accordance with ISO 1856, method A, elongation at break in the range of above 50%, determined in accordance with PN-EN ISO 1798, tensile strength in the range of above 40kPa, determined in accordance with PN-EN ISO 1798.
